# EUROPÄISCHE PATENTSCHRIFT

(11) **EP 0 969 766 B1**
(45) Veröffentlichungstag und Bekanntmachung des Hinweises auf die Patenterteilung: **23.03.2005**
(21) Anmeldenummer: 97954711.4
(22) Anmeldetag: 23.12.1997
(51) Int. Cl.: A61B 17/00

(54) **ENDOSKOPISCHES INSTRUMENT**
ENDOSCOPIC INSTRUMENT
INSTRUMENT ENDOSCOPIQUE

(30) Priorität: 03.01.1997 DE 19700114
(43) Veröffentlichungstag der Anmeldung: 12.01.2000
(73) Patentinhaber: KLINIKUM DER ALBERT-LUDWIGS-UNIVERSITÄT FREIBURG, D-79106 Freiburg (DE)
(72) Erfinder: MATERN, Ulrich, D-79283 Bollschweil (DE); WALLER, Peter, D-82131 Gauting (DE)
(74) Vertreter: Lohr, Georg, Dr.
(86) Internationale Anmeldenummer: PCT/DE1997/003019
(87) Internationale Veröffentlichungsnummer: WO 1998/029024

(56) Entgegenhaltungen:
- WO-A-95/10230
- DE-A- 3 630 210
- DE-A- 4 228 909
- DE-A- 4 429 812
- DE-A- 4 431 561
- DE-C- 4 216 874
- US-A- 2 518 994

## Beschreibung

### Technisches Gebiet

Die Erfindung bezieht sich auf einen Griffteil eines endoskopischen Instrumentes mit wenigstens einem beweglichen Bedienelement zur mechanischen Ansteuerung einer am distalen Schaftende des endoskopischen Instrumentes angebrachten Werkzeugeinheit, und einem Grundkörper.

### Stand der Technik

Der Erfolg des Einsatzes endoskopischer Instrumente in der Chirurgie hängt nicht zuletzt von der Bedienfreundlichkeit derartiger Instrumentarien ab, die in sehr starkem Maße durch die ergonomische Ausbildung der Griffteile bestimmt ist.

So sind beispielsweise seit langem sogenannte Scherengriffe bekannt, die durch Betätigung das Öffnen und Schließen von an endoskopischen Instrumenten distalseitig angebrachten Zangenwerkzeugen erlauben. Sind distalseitig Verdrehungen der Zangenwerkzeuge in offener oder geschlossener Stellung erforderlich, so muß der Operateur bei derartigen, scherenartig ausgebildeten Griff teilen, das Handgelenk entsprechend verdrehen. Dies ist jedoch nur bis zu einem bestimmten Winkel möglich, der durch die maximale Verdrehbarkeit des menschlichen Handgelenkes vorgegeben ist. Ein weiteres Verdrehen ist durch die Rotation des Schaftes mit Hilfe einer Rändelmutter möglich. Ferner können Branchengriffe und Scherengriffe nicht der individuellen Handgröße angepasst werden.

In der DE 42 16 874 C1 ist eine Kupplung für chirurgische Instrumente offenbart. Diese Kupplung weist einen kugelförmigen Griff auf, welcher in der Handfläche des Chirurgen liegt. Weiterhin sind Greifelemente zur Betätigung mit den Fingern vorgesehen. Das Griffteil kann mit dem Instrument durch eine besonders ausgestaltete Kupplung mit Riegelstücken formschlüssig verbunden werden.

### Darstellung der Erfindung

Der Erfindung liegt die Aufgabe zugrunde, ein Griffteil eines endoskopischen Instrumentes mit wenigstens einem beweglichen Bedienelement zur mechanischen Ansteuerung einer am distalen Schaftende des endoskopischen Instrumentes angebrachten Werkzeugeinheit und einem Grundkörper, derart ergonomisch auszugestalten, so daß das Griffteil sowohl für den linkshändigen als auch rechtshändigen Einsatz geeignet ist. Das Griffteil ist möglichst in der räumlichen Geometrie einfach auszugestalten, so daß die damit verbundenen Herstellkosten reduziert werden können. Darüberhinaus sollen individuelle Anpaßmaßnahmen am Griffteil möglich sein, um den unterschiedlichen Handgrößen der Operateure gerecht zu werden.

Die Lösung der der Erfindung zugrundeliegenden Aufgabe ist im Patentanspruch 1 angegeben. Vorteilhafte Ausgestaltungen der Erfindung sind Gegenstand der Unteransprüche.

Erfindungsgemäß ist ein Griffteil eines endoskopischen Instrumentes mit wenigstens einem beweglichen Bedienelement zur mechanischen Ansteuerung einer am distalen Schaftende des endoskopischen Instruments angebrachten Werkzeugeinheit und einem Grundkörper derart ausgestaltet, daß der Grundkörper eine weitgehend symmetrische Form aufweist, die der Ergonomie der menschlichen Hand derart angepaßt ist, daß das endoskopische Instrument allein über den Grundkörper, der zumindest teilweise von einer zu einer Faust geformten Handinnenfläche umschließbar ist, fixierbar ist, und daß das Bedienelement relativ zum Grundkörper bewegbar ist, dadurch gekennzeichnet, dass Rändelschrauben vorgesehen sind, mit denen zum einen die Verdrehung der Werkzeugeinheit samt Instrumentenschaft um die Schaftachse möglich ist und zum anderen eine feste Verbindung des Instrumentenschaftes mit dem Griffteil hergestellt werden kann.

Der Erfindung liegt die Idee zugrunde, den Grundkörper des Griffteils möglichst symmetrisch auszugestalten und in einer Größe auszuführen, so daß der Grundkörper weitgehend ausschließlich durch eine zu einer Faust geformten Handinnenfläche gehalten werden kann. Vorteilhafte Geometrien derartiger Grundkörper sind sphärische Körper, wie beispielsweise eine Kugel, oder aber länglich ausgebildete Formen, die vorzugsweise der Handflächenbreite entspricht und von dieser in der gekrümmten Haltung weitgehend umfaßt werden können.

Durch die erfindungsgemäße symmetrische Ausgestaltung des Grundkörpers ist es für den Operateur möglich, den Griffteil sowohl mit der linken als auch mit der rechten Hand zu greifen. Insbesondere bei der Durchführung von chirurgischen Operationen kann es vorkommen, daß der Operateur mehrere endoskopische Instrumente gleichzeitig zu bedienen hat; hierbei ist es von großem Vorteil, wenn der Operateur ein und das gleiche endoskopische Instrument sowohl mit der linken als auch mit der rechten Hand bedienen kann, um gegebenenfalls die Operationen schneller und damit für den Patienten schonender durchführen zu können.

Der Grundkörper ist vorzugsweise in proximaler Verlängerung zur Instrumentenachse angebracht und relativ zum Bedienelement verschiebbar zu fixieren. Auf diese Weise ist es möglich, den Griffteil individuell an die Größe der Operateurshand anzupassen.

Zwar ist es möglich, das gesamte endoskopische Instrument allein mit der Handinnenfläche, die den Grundkörper zu umfassen vermag, zu halten, doch ist vorzugsweise in distaler Richtung dem Grundkörper ein Gegenhalt angebracht, mit der der Operateur wenigstens einen seiner Finger zum Gegenhalt anlegen kann. Das Bedienelement ist vorzugsweise beweglich an dem Gegenhalt angebracht.

### Kurze Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: eine schematisierte Darstellung eines Griffteils für ein endoskopisches Instrument,
- Fig. 2: alternative Ausführungsform für einen Grundkörper.

### Beschreibung eines Ausführungsbeipsiels

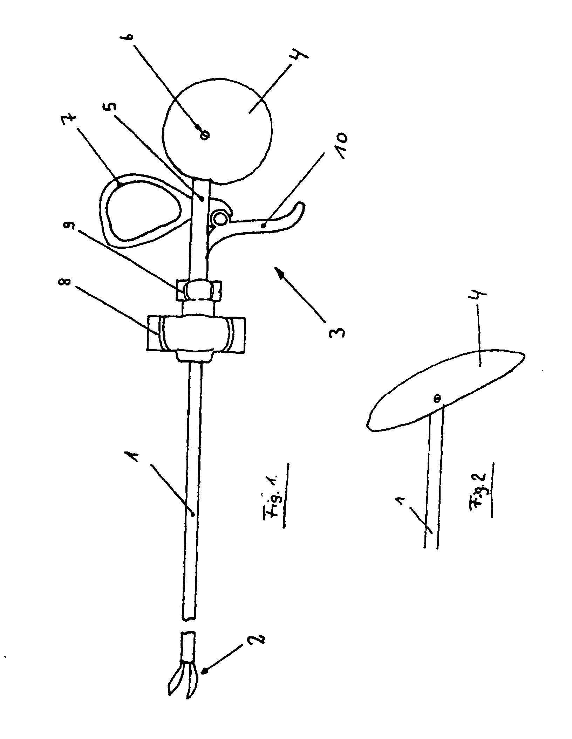

In Fig. 1 ist ein endoskopisches Instrument mit einer Instrumentenschaftachse 1 sowie eine distalseitig an den Schaft 1 angeordnete Werkzeugeinheit 2 dargestellt, die im Falle des Ausführungsbeispiels eine Greifeinheit darstellen soll. Proximalseitig an dem Intrumentenschaft 1 ist ein Griffteil 3 angeordnet, mit dem es möglich ist, die zangenartige Werkzeugeinheit zu öffnen und zu schließen sowie um die Instrumentenschaftachse zu verdrehen. Bevorzugt sieht das Griffteil 3 einen Grundkörper 4 vor, der in Art einer Kugel ausgebildet ist und der sowohl von der linken als auch rechten Hand des Operateurs wenigstens derart mit der Handinnenfläche umschlossen werden kann, so daß das gesamte endoskopische Instrument durch den Grundkörper 4 getragen werden kann. Der Grundkörper 4 ist proximalseitig auf einer Verlängerung 5 angebracht und kann relativ zur Verlängerung 5 verschoben und mit einer Fixiereinheit 6, die z.B. in Art einer Madenschraube ausgebildet sein kann, auf der Verlängerung 5 fixiert werden. Durch diese Verschiebbarkeit ist es möglich, den Grundkörper 4 relativ zu dem Bedienelement 7 derart einzustellen, daß es für einen Operateur ergonomisch bequem ist, das Griffteil 3 zu bedienen. Zur Erhöhung der Haltekraft ist ein Haltesteg 10 vorgesehen, an dem wenigstens ein Finger anlegbar ist, um den Gegendruck des Grundkörpers 4 in Richtung der Handinnenfläche eines Operateurs, die in der Zeichnung nicht dargestellt ist, zu erhöhen.

Durch Bewegen des Bedienelementes 7 relativ zum Grundkörper 4 kann die Werkzeugeinheit 2 geöffnet und geschlossen werden. Zusätzlich sind Rändelschrauben 8 und 9 vorgesehen, mit denen zum einen die Verdrehung der Werkzeugeinheit 2 samt Instrumentenschaft 1 um die Schaftachse möglich ist und zum anderen eine feste Verbindung des Instrumentenschaftes mit dem Griffteil 3 hergestellt werden kann.

In Fig. 2 ist eine alternative Ausführungsform eines Grundkörpers 4 angegeben, der im Querschnitt eine Keilform aufweist und ergonomisch an die menschliche Handinnenfläche angepaßt ist. Auch mit dieser Form ist es möglich, das gesamte endoskopische Instrument nur durch Umschließen des Griffteils 4 zu halten.

## Patentansprüche

1. Endoskopisches Instrument mit einem Griffteil (3) mit wenigstens einem beweglichen Bedienelement (7) zur mechanischen Ansteuerung einer am distalen Schaftende des endoskopischen Instrumentes angebrachten Werkzeugeinheit (2) und einem Grundkörper (4), welcher eine weitgehend symmetrische Form dergestalt aufweist, dass das endoskopische Instrument allein über den Grundkörper (4), der zumindest teilweise von einer zu einer Faust geformten Handinnenfläche umschließbar ist, fixierbar ist, und dass das Bedienelement (7) relativ zum Grundkörper (4) bewegbar ist,
**dadurch gekennzeichnet, dass** Rändelschrauben (8, 9) vorgesehen sind, mit denen zum einen die Verdrehung der Werkzeugeinheit (2) samt Instrumentenschaft (1) um die Schaftachse möglich ist und zum anderen eine feste Verbindung des Instrumentenschaftes mit dem Griffteil (3) hergestellt werden kann.

2. Endoskopisches Instrument nach Anspruch 1, **dadurch gekennzeichnet, dass** der Grundkörper (4) die Form einer Kugel aufweist.

3. Endoskopisches Instrument nach Anspruch 1, **dadurch gekennzeichnet, dass** der Grundkörper (4) keilförmig ausgebildet ist, der von der Handinnenfläche weitgehend vollständig umschließbar ist.

4. Endoskopisches Instrument nach einem der Ansprüche 1 bis 3, **dadurch gekennzeichnet, dass** der Grundkörper (4) in Verlängerung zur Instrumentenschaftachse angebracht ist und entlang dieser verschiebbar ist, sowie mittels einer Fixiervorrichtung an der Instrumentenschaftachse (1) fixierbar ist.

5. Endoskopisches Instrument nach einem der Ansprüche 1 bis 4, **dadurch gekennzeichnet, dass** am Instrumentenschaft ein Haltesteg (10) vorgesehen ist.

6. Endoskopisches Instrument nach Anspruch 5, **dadurch gekennzeichnet, dass** der Haltesteg (10) als Gegengriff für den Grundkörper (4) dient.

## Claims

1. Endoscopic instrument comprising a handle portion (3) with at least one movable operating element (7) for mechanically controlling a tool unit (2) mounted to a distal shaft end of the endoscopic instrument, and a base body (4) that is substantially symmetrically formed to be of such shape that the endoscopic instrument can be localized via only the base body (4) that can be encompassed at least partially by the palm of a hand when forming a fist, and that the operating element (7) is movable relative to the base body (4),
**characterized in that** knurled screws (8, 9) are provided to enable, on the one hand, a rotation of the tool unit (2) including the instrument shaft (1) about the shaft axis, and on the other hand, the effecting of a firm connection of the instrument shaft with the handle portion (3).

2. Endoscopic instrument according to claim 1, **characterized in that** the base body (4) has the shape of a sphere.

3. Endoscopic instrument according to claim 1, **characterized in that** the base body (4) is formed to be wedge-shaped, and can be encompassed substantially completely by the palm of a hand.

4. Endoscopic instrument according to any one of claims 1 to 3, **characterized in that** the base body (4) is mounted as an extension of the instrument shaft axis along which it is displaceable, and is also adapted to be locked to the instrument shaft axis (1) by means of a locking device.

5. Endoscopic instrument according to any one of claims 1 to 4, **characterized in that** a holding bracket (10) is provided on the instrument shaft.

6. Endoscopic instrument according to claim 5, **characterized in that** the holding bracket (10) serves as a counter-handle for the base body (4).

## Revendications

1. Instrument endoscopique à un élément de manette (3), comprenant au moins un élément de manoeuvre mobile pour le contrôle mécanique d'une unité d'outil (2) disposé à l'extrémité distale de la tige de l'instrument endoscopique, et un corps de base (4), qui a une forme largement symétrique d'une telle façon, que l'instrument endoscopique se puisse fixer seulement via ledit corps de base (4), qui peut être entouré, au moins en partie, par une paume intérieure de la main serrée en poing, et que ledit élément de manoeuvre (7) soit mobile relativement audit corps des base (4),
**caractérisé en ce que** des vis moletées (9) sont fixées, moyennant lesquelles, d'un côté, la rotation de ladite unité d'outil (2), y compris ladite tige de l'instrument (1), autour de l'axe de la tige devient possible et, d'autre côté, une connexion solide peut être établie entre ladite tige de l'instrument et ledit élément de manette (3).

2. Instrument endoscopique selon la revendication 1, **caractérisé en ce que** ledit corps de base (4) a la forme d'une sphère.

3. Instrument endoscopique selon la revendication 1, **caractérisé en ce que** ledit corps de base (4) a une configuration cunéiforme et peut être entouré, largement complètement, par la paume intérieure de la main.

4. Instrument endoscopique selon une quelconque des revendications 1 à 3, **caractérisé en ce que** ledit corps de base (4) est monté dans la prolongation de l'axe de la tige de l'instrument et déplaçable le long de cet axe, et se peut fixer moyennant un dispositif fixateur audit axe (1) de la tige de l'instrument.

5. Instrument endoscopique selon une quelconque des revendications 1 à 4, **caractérisé en ce qu'**une traverse de maintien (10) est disposé à la tige de l'instrument.

6. Instrument endoscopique selon la revendication 5, **caractérisé en ce que** ladite traverse de maintien (10) sert en tant que contre manette pour ledit corps de base (4).
